# EUROPEAN PATENT APPLICATION

(11) **EP 2 336 108 A1**
(43) Date of publication of application: **22.06.2011**
(21) Application number: 09809368.5
(22) Date of filing: 27.08.2009
(51) Int. Cl.: C07D 207/34, C07K 7/00, A61K 31/4025, A61P 35/00, A61P 31/00

(54) **HYBRID TRIPYRROLE-OCTAARGININE COMPOUNDS AND THEIR USE AS MEDICAMENT IN THE TREATMENT OF CANCER AND MICROBIAL ILLNESSES**

(30) Priority: 29.08.2008 ES 200802520
(71) Applicant: Universidade De Santiago De Compostela, 15782 Santiago de Compostela (ES)
(72) Inventor: VAZQUEZ VAZQUEZ, Olalla, E-15782, Santiago de Compostela (ES); VAZQUEZ SENTIS, Eugenio, E-15782, Santiago de Compostela (ES); BLANCO CANOSA, Juan Bautista, E-15782, Santiago de Compostela (ES); MARTINEZ COSTAS, Jose, E-15782, Santiago de Compostela (ES); MASCAREÑAS CID, Jose Luis, E-15782, Santiago de Compostela (ES); CASTEDO EXPOSITO, Luis, E-15782, Santiago de Compostela (ES)
(74) Representative: Pons Ariño, Angel
(86) International application number: PCT/ES2009/070356
(87) International publication number: WO 2010/023346

(57) **Abstract**

Hybrid tripyrrole-octaarginine compounds. Hybrids formed of a tripyrrole group linked to a polyarginine peptide which permit efficient cellular internalisation and promotes greater affinity for DNA. The method of obtainment and uses thereof.

## Description

The present invention relates to a compound formed by a tripyrrole group bound to a polyarginine peptide. Furthermore, it relates to the method of obtainment and uses thereof, since said compound permits the efficient cellular internalization and promotes a greater affinity for the DNA.

### PRIOR ART

One of the molecular strategies of greatest interest for the development of antibiotic or antitumoral agents is based on the interaction of determined molecules with specific areas of the DNA (L. Strekowski, Mutation Res. 2007, 623, 3-13)

Some of these molecules are in clinical trial phase and others, such as anthracyclines or bleomycin, have already been used for several years in clinical practice.

Most of the DNA minor groove-binding ligands (MGBLs) have difficulty in crossing cell membranes and efficiently reaching the cell nucleus. Thus, for example, polyamides based on the antibiotic Distamycin A, despite the fact that they are capable of interacting with the specific sequences of the DNA, undergo very slow cellular internalization processes and dependent on their structure. (N.G. Nickols, C.S. Jacobs, M.E. Farkas, P.B. Dervan, Nucleic Acids Res. 2007, 35, 363-370; Edelson, T.P. Best, B. Olenyuk, N.G. Nickols, R.M. Doss, S. Foister, A. Heckel, P.B. Dervan, Nucleic Acids Res. 2004, 32, 2802-281)).

In this sense, some documents of the state of the art, for example patent application W02008043366, that relate to compounds for the transport of biological agents through a membrane. Some of these compounds are cationic peptides.

### EXPLANATION OF THE INVENTION

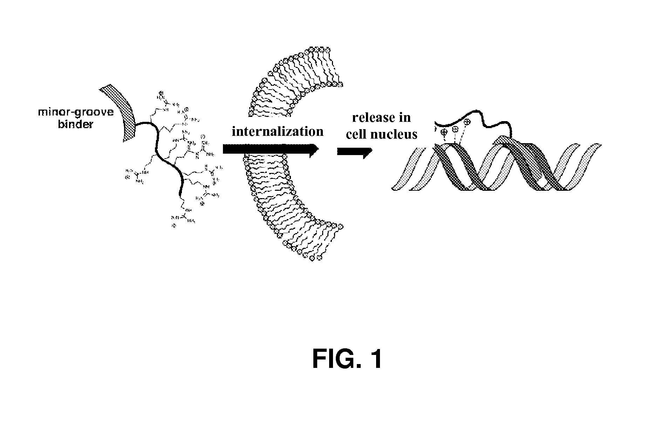

The present invention aims to resolve the problem of the difficulty of certain agents that interact with DNA in crossing cell membranes and rapidly reaching the cell nucleus, as well as improving the interaction with the DNA without loss of selectivity. Therefore, the present invention relates to the conjugation of a recognition element of the minor groove of the DNA of tripyrrole type with polyarginine fragments with the aim of obtaining agents that interact with DNA "in vivo" quickly and efficiently (FIG 1).

With the aim of studying structure-function relations, a series of peptide-tripyrrole peptides were prepared, as well as controls without the tripyrrole and without the polyarginine peptide, and a fluorophore group was incorporated in the molecules to be able to monitor the cellular internalization process by analysis with a fluorescence microscope. The internalization assays were performed in live HeLa cells.

The results, provided in the examples section, demonstrate how the conjugation of a tripyrrole to octaarginines permit an efficient cellular internalization and a very fast localization of the cell nucleus. The presence of the basic peptide region causes a very considerable increase in affinity of the system by DNA sequences rich in adenines (over 100 times with respect to tripyrrole alone). The functioning of these conjugates is based on the synergy between the specific recognition element (tripyrrole) and the peptide fragment, which not only facilitates cell transport but also promotes a greater affinity for the DNA.

Therefore, a first aspect of the present invention relates to a compound comprising a recognition element of the minor groove of the DNA of tripyrrole type bound to a polyarginine peptide, more specifically octaarginine ((Arg)₈). This bond is produced by means of a spacer group comprising, at least, a lysine (Lys) whereby the tripyrrole is bound. (From hereon we will call them compounds of the invention).

As recognition element of the minor groove of the DNA or DNA minor groove-binding ligands (or MGBLs) a series of compounds are known that exhibit a series of antitumoral, antimicrobial, antiviral and antiprotozoal properties. It is known that the structures that contain an imidazole and a pyrrole, of the type of the heterocyclic polyamide Distamycin may bind to the minor groove of the double strand, preferably in AT-rich areas, increasing the twisting of the double strand, inducing a positive supercoiling and therefore interfering both in the replication and in the transcription. The bond to the DNA is due in part to the fact that the amide hydrogens of the N-methylpyrrolecarboxamides form bifurcate hydrogen bridges with the N₃ atoms of adenine and O₂ of the thymidine in the minor groove (Koopka et al., 1985. Proc. Natl. Acad. Sci. 82:1376; Koopka et al., 1985. J. Mol. Biol. 183:553). The pyrrole rings completely fill the groove, excluding the amine group of the guanine of the G,C base pairs, whilst van der Waals forces are established with the walls of the groove, showing affinity for the sequences rich in A and T (Taylor et al., 1985. Tetrahedron 40:457; Schultz & Dervan. 1984. J. Biomol. Struct. Dyn. 1:1133).

With the aim of studying the internalization and the bond of this complex with the DNA, it was bound to a fluorophore group, so that in a preferred embodiment of the invention, this compound may also be bound to a fluorophore group, preferably fluorescein (Flu).

In another preferred embodiment of the present invention, the tripyrrole has the general formula (I):

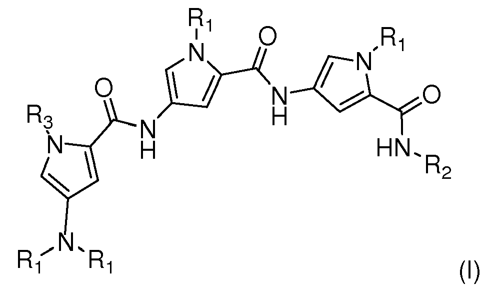

Where, R₁ is a substituent that can be the same as or different in each case and is independently selected from the list comprising H, an alkyl group (C₁-C₆) or an acyl group; R₂ is a substituted alkyl group of general formula (IIA): 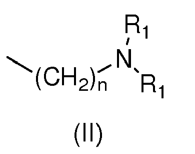 or of general formula (IIB):

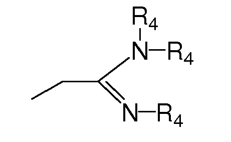

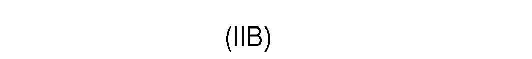

Where R₁ is defined as above and n takes values between 1 and 6, preferably 3, 4 and 5, and R4 is a substituent that can be the same or different in each case and d is independently selected from the list comprising H, or an alkyl group (C₁-C₆), preferably a methyl.
R₃ is a substituted alkyl group of general formula (III): 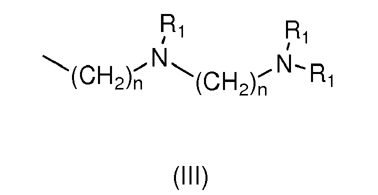 Where R₁ and n are defined as above.

In an even more preferred embodiment, R₁ is the same or different in each case and is independently selected from the list comprising H, an alkyl group (C₁-C₃), preferably methyl or an acetyl group. More preferably the tripyrrole compound is the following: 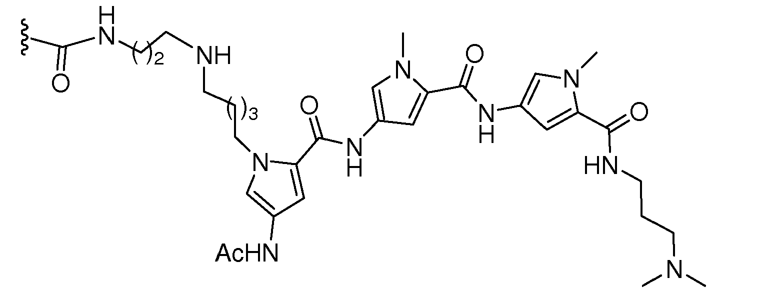 Where 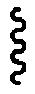 represents the binding site with the lysine of the spacer.

The term "alkyl" cited as substituent of R₁ relates in the present invention to aliphatic, linear or branched chains, which have from 1 to 6 carbon atoms. Preferably the alkyl group has between 1 and 3 carbon atoms, for example, methyl, ethyl or n-propyl.

As regards the spacer of the present invention, it is a structure composed of at least one lysine (Lys) amino acid, whereto the tripyrrole and 6-aminohexanoic acid (Ahx) groups are bound, where the peptide and/or the fluorophore will be bound if this is included.

In a preferred embodiment, the spacer is of the type (Lys-Ahx) or (Ahx-Lys-Ahx), the latter having the following formula (IV):

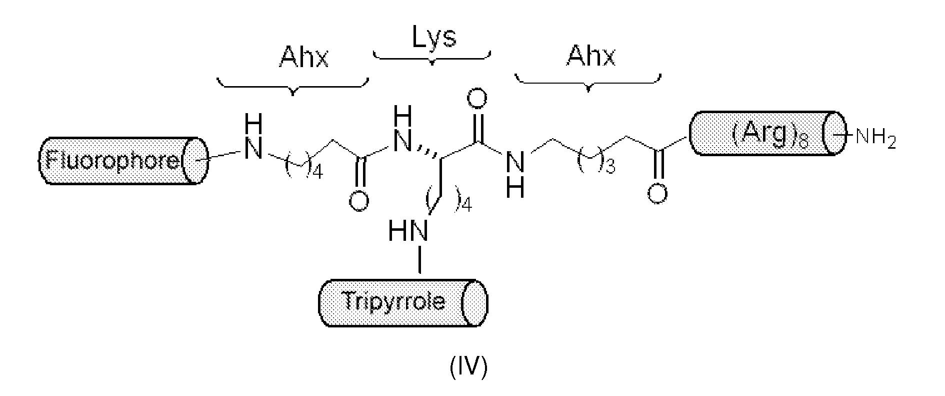

In a more preferred embodiment, the compound is that of formula 6A or any of its salts or isomers:

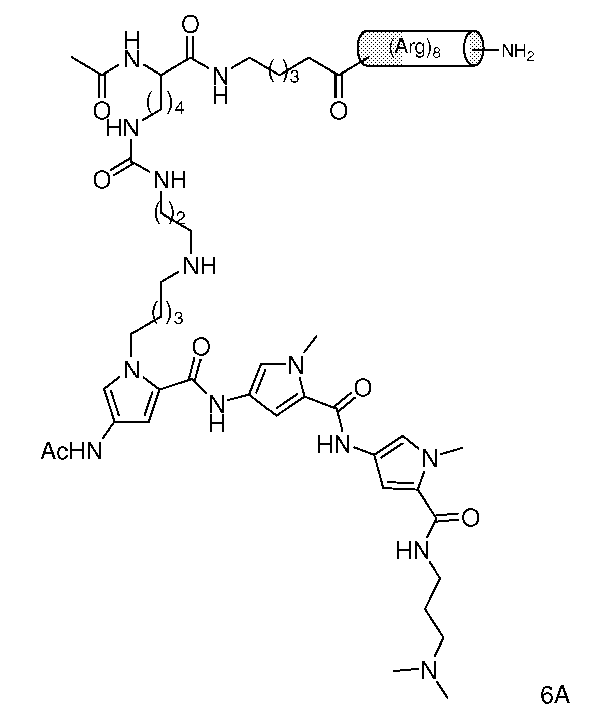

In another more preferred embodiment, the compound is that of formula 1A or any of its salts or isomers, similar al compound 6A, and which incorporates the fluorophore group in its structure:

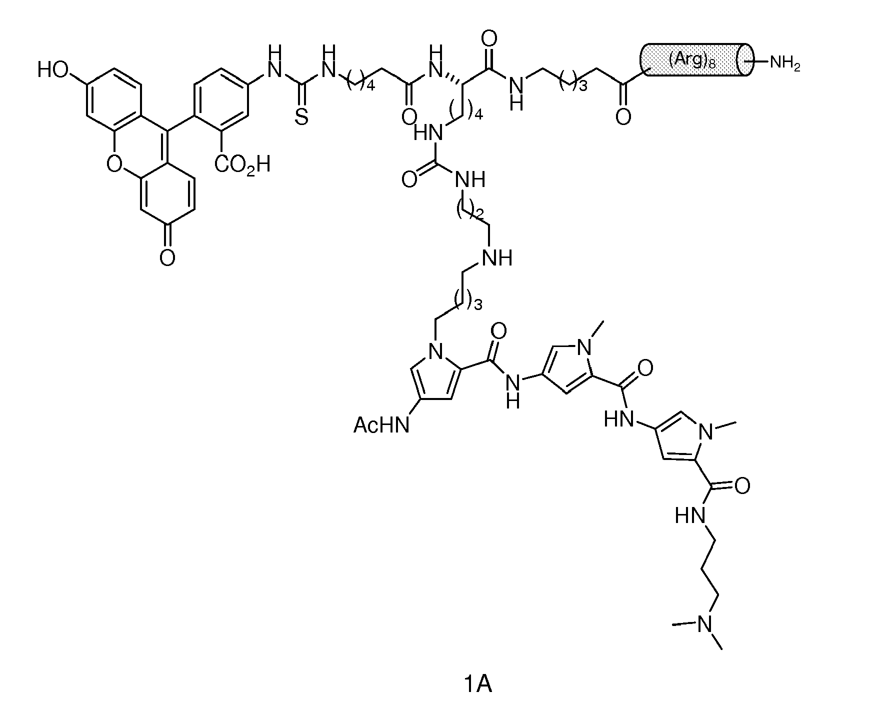

The synthesis of these molecules is performed using solid phase methodologies, for which reason another aspect of the invention relates to a process to obtain the described compounds, comprising the following steps:
a) synthesis of the peptides by Fmoc methods in solid phase (fmoc-peptides)
   - temporary deprotection of the Fmoc group
   - coupling of the amino acids
   - selective deprotection of the lysine residue
   - fractioning and deprotection
b) synthesis of the tripyrrole-polyarginine compounds
   - Coupling of the amino tripyrrole to the peptide, whilst this is still bound to the solid phase and protected.
   - Elimination of the protector groups and separation of the solid resin 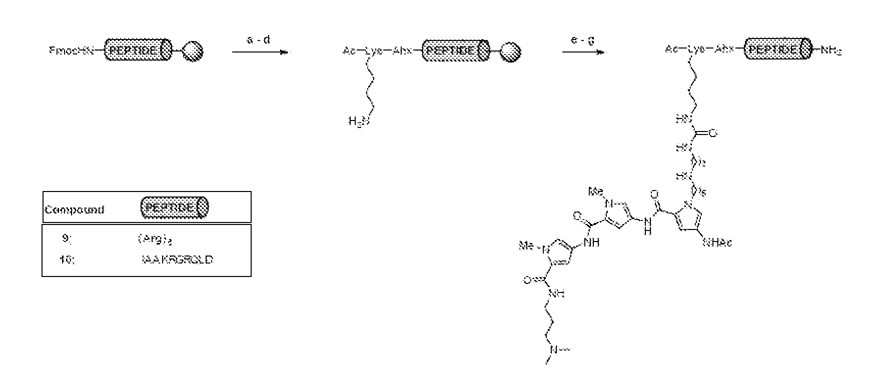 Diagram of general synthesis of compounds without fluorophore

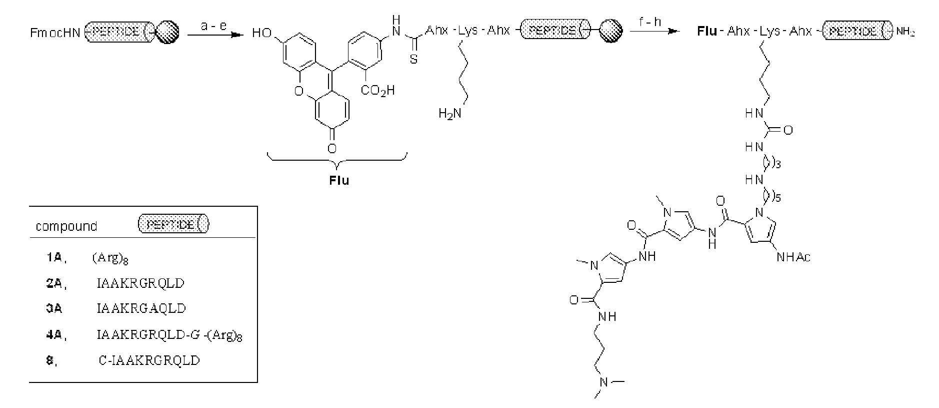

### Diagram of general synthesis of compounds with fluorophore

The DNA minor groove binder (MGBLs) compounds constitute an important class of derivatives in anti-tumour therapy, being of intrinsic interest due to their biological and medical properties. Furthermore, the recognition strategy could be applicable for the obtainment of new regulatory agents of genetic processes with clinical possibilities.

Thus another aspect of the present invention relates to a pharmaceutical composition comprising at least one compound of the invention, as previously described, or any of its combinations, in a pharmaceutically effective quantity, or a pharmaceutically acceptable salt, prodrug, solvate or stereoisomer, for administration to a patient. The pharmaceutically acceptable adjuvants and vehicles that can be used in said compositions are the adjuvants and vehicles known by persons skilled in the art and typically used in the preparation of therapeutic compositions.

In the sense used in this description, the expression, "therapeutically effective quantity" relates to the agent or compound capable of developing the therapeutic action determined by its pharmacological properties, calculated to produce the desired effect and, in general, it will be determined, among other causes, by the characteristics typical of the compounds, including, age, condition of the patient, severity of the alteration or disorder and the route of administration and frequency.

The solutions for injection or intravenous infusion may contain as vehicle, for example, sterile water or, preferably, they may be in the form of sterile isotonic saline solutions. The suspensions or solutions for intramuscular injections may comprise, together with the active compound, a pharmaceutically acceptable vehicle, for example, sterile water, olive oil, ethyl oleate, glycols, for example propylene glycol, and, if desired, a suitable quantity of lidocaine hydrochloride.

In the forms for topical application, for example, creams, lotions or pastes for use in dermatological treatment, it is possible to mix the active component with oleaginous excipients or conventional emulsifiers.

The solid oral forms, for example tablets and capsules, may contain together with the active compound, diluents, for example lactose, dextrose, sucrose, cellulose, corn starch and potato starch; lubricants, for example silica, talc, stearic acid, magnesium and/or calcium stearate and/or polyethylene glycols; ligand agents, for example starches, gum arabics, gelatine, methylcellulose, carboxymethylcellulose and polyvinylpyrrolidone; disintegrating agents, for example starch, aginic acid, alginates and sodium glycolate and starch; effervescent mixtures; colouring agents; sweeteners; wetting agents, for example lecithin, polysorbates and laurylsulfates, and, in general, non-toxic and pharmacologically inactive substance used in the pharmaceutical formulation. Said pharmaceutical preparation may be manufactured by known techniques, for example by means of mixing, granulation, tablet formation, sugar coating or film coating processes.

In another particular embodiment said therapeutic composition is prepared in solid or aqueous suspension form, in a pharmaceutically acceptable diluent. The therapeutic composition provided by this invention can be administered by any appropriate route of administration, for which purpose said composition will be formulated suitable for the chosen administration route.

The use of the compounds of the invention is compatible with their use in protocols wherein the compounds of the invention, or their mixtures are used by themselves or in combinations with other treatments or any medical process.

Therefore, another aspect of the present invention relates to the use of any of the compounds described of the pharmaceutical compositions that are included for the preparation of a drug.

The compounds of the invention are useful as antineoplastic and antimicrobial agents. They particularly show cytostatic properties towards the tumour cells, so that they can be useful for inhibiting the growth of certain tumours in mammals, including humans, such as for example, carcinomas, such as breast carcinoma, lung carcinoma, bladder carcinoma, colon carcinoma and ovarian and endometrial tumours. Other neoplasias wherein the pharmaceutical compositions of the present invention may find application are, for example, sarcomas, such a soft tissue and bone sarcoma and haematological malignancies, such as, for example, leukaemias. Hence, a preferred embodiment of this aspect of the invention, is the use of any of the compounds described or of the pharmaceutical compositions that are included for the preparation of a drug for the treatment of cancer.

Part of this preferred embodiment is a combined method to treat cancer or improve the conditions of mammals, including humans, that suffer from cancer, whose method consists of administering at least one compound of the invention or a pharmaceutically acceptable salt thereof and an additional antitumoral agent, sufficiently close in time and in sufficient quantities to produce a therapeutically useful effect.

Antineoplastics are substances that prevent the development, growth and/or proliferation of malignant tumour cells.

The term "antitumoral agent" aims to include both the antitumoral drug and "cocktails", i.e. a mixture of said drugs, according to clinical practice.

The compounds of the invention also show considerable efficacy in interference with the reproduction activity of pathogenic viruses and protect the tissue cells from viral infections. For example, they show activity against DNA virus such as, for example, herpes, for example herpes simplex virus and herpes zoster virus, vaccinia virus, RNA virus such as, for example, *Rhinovirus* and *Adenovirus,* and against retrovirus such as, for example, sarcoma virus, for example murine sarcoma virus and leukaemia virus, for example Friend's leukaemia virus. Hence, another preferred embodiment of this aspect of the invention, is the use of any of the compounds described or the pharmaceutical compositions that include them for the preparation of a drug for the treatment of microbial diseases. An even more preferred embodiment of the invention is the use of any of the compounds described or the pharmaceutical compositions that include them for the preparation of a drug for the treatment of viral microbial diseases.

Therefore, and endorsed by the results obtained and stated in the examples section, it can be stated that an efficient entry occurs in the cell nucleus of the compounds, conjugates or hybrids containing the octaarginine and the tripyrrole covalently bound. (It is represented illustratively in FIG.1)

Furthermore, the tripyrrole system, by permitting a strong interaction with the DNA facilitates the anchoring of the hybrids in the nucleus chromosomes. In other words, rapid intranuclear accumulation, in particular of the compounds of formula 1A (tripryrrole-oligoarginine), is due to the cooperative action of both fragments, oligoarginine and the bonding molecule to the minor groove of the DNA (tripyrrole).

Furthermore, the interaction is selective for DNA sites containing at least 4 adenines or thymines following one another.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following figures and examples are provided by way of illustration, and are not intended to limit the present invention.

### BRIEF DESCRIPTION OF THE FIGURES

**FIG. 1**.- Shows the functioning of the conjugates of tripyrroles with polyarginines to induce cellular internalization and the increase in affinity.
**FIG. 2**.- Shows the intracellular distribution of the different hybrids in HeLa cells. The exposure times were different for each sample due to the variations observed in the total emission as a result of the different internalization efficiencies. The exposure times were: 1 A, 1 B, 4A = 1 /18 s; 2A = 1/2 s; 5A = 1/6 s; 3A = 1 s.
**FIG. 3**. PAGE of the interaction of the peptide **6** with a double-stranded oligonucleotide with the sequence ATTTT. Lanes 1-10: [**6**]= 0, 2, 4, 6, 8, 10, 12, 15, 20, 25 nM. Ahx = 6-aminohexanoic.

### DETAILED EXPLANATION OF EMBODIMENTS

The invention will be illustrated below with assays performed by the inventors, which reveals the specificity and efficacy of the compounds of the invention.

### EXAMPLE 1.- Preparation of the tripyrrole-peptide compound and internalization assays.

In order to study structure-function relations, a series of peptide-tripyrrole hybrids **1A-5A** were prepared, as well as controls without the tripyrrole (**1B-4B**). A fluorophore group was incorporated in the molecules to be able to monitor the cellular internalization process by analysis with a fluorescence microscope. The synthesis of the molecules was performed using solid phase methodologies.

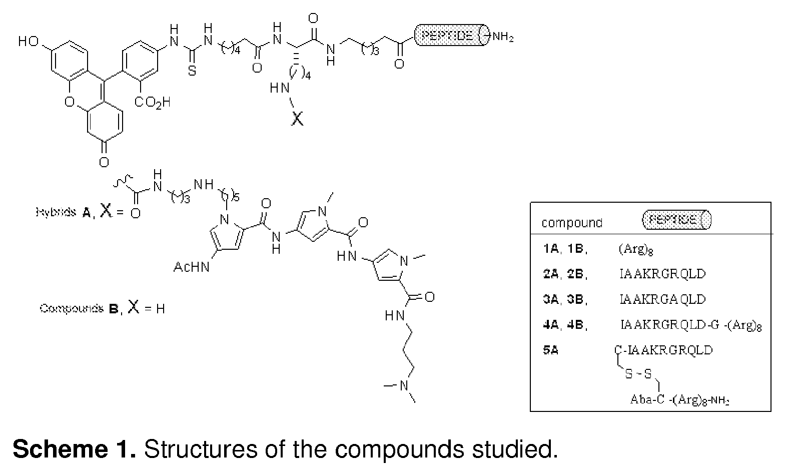

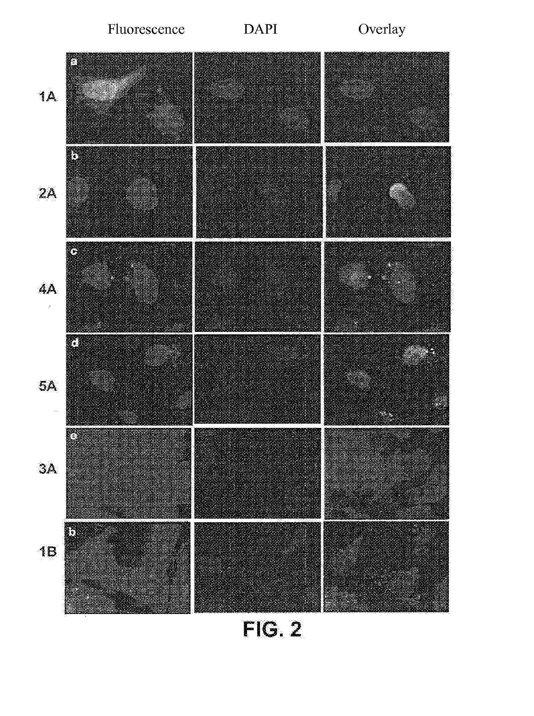

The internalization assays were performed in live HeLa cells, recording the fluorescence images after 30 min, 90 min and 3 h. As shown in Figure 2 (FIG. 2), the incubation of cells with the tripyrrole-octaarginine conjugate **1A** during 90 min at 37 ºC gave rise to a homogeneous and intense fluorescence signal in the cell nucleus. Later, it was observed that said signal was already observed after 30 minutes. This demonstrates that the internalization is very fast. An internalization control of the same tripyrrole without the octaarginine sequence demonstrated that no intracellular fluorescence was observed even after 3 h. It therefore seems clear that octaarginine is vital for an efficient transport of the conjugate.

In the case of hybrid **2A** containing as peptide fragment a nuclear localization signal (NLS) instead of polyarginine, the fluorescence signal observed after the incubation is also localized in the cell nucleus; however, the signal intensity is less than in the case of hybrid **1A**, probably due to the lower cytoplasmic internalization capacity inherent to this sequence. The introduction of an octaarginine sequence, in addition to the NLS, either linearly (**4A**) or through a labile disulfide bond (**5A**), improved the internalization properties with respect to the hybrid that exclusively contains NLS (**2A**); however, the transport capacity was in both cases less than that of the hybrid that exclusively contained octaarginine **1A**. Conjugate **3A**, similar to **2A** but with a mutation in the NLS was not sufficiently internalized, a revealed by the low fluorescence observed inside the cells. The product appears uniformly distributed by the cellular cytoplasm, which agrees with the loss of capacity of nuclear signalling of the mutated NLS.

The control peptides that do not have the tripyrrole fragment are not capable of reaching the cell nucleus. Particularly relevant is the case of peptide **1B**, which is analogous to **1A**, but without the tripyrrole. As is observed in Figure 2 (FIG. 2), the fluorescence is intense, but is exclusively localized in the cytoplasm.

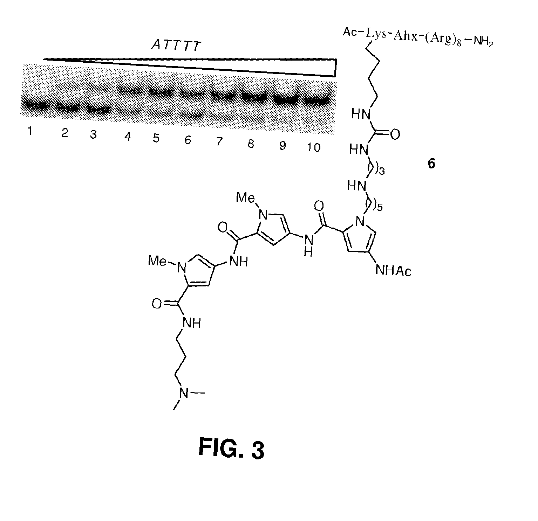

On the other hand, to verify the possible involvement of the fluorophore, hybrid **6** was used, similar to **1A** but without the fluorophore group. This showed an affinity for DNA sequences rich in adenine, around 6 nM at ambient temperature (FIG. 3). Furthermore, the interaction is selective for DNA sites containing at least 4 adenines or thymines one after another.

### EXAMPLE 2.- Synthesis and purification of the peptides

The peptides were manually synthesized in a scale of 0.1 mmol following the standard peptide synthesis protocol following the Fmoc/tBu strategy. A PAL-PEG (0.19 mmol/g) resin as used for the synthesis. Each amino acid was activated during 2 min in DMF before its addition to the deprotected resin. The peptide bond formation reaction was left to take place for a minimum of 45 min until the TNBS test gave negative. The deprotection of the temporary Fmoc group was carried out by treatment of the resin with 20% piperidine in DMF during 15 min. The final peptides were obtained after the deprotection/release of the resin by treatment with the standard deprotection cocktail with TFA as specified below.

*Deprotection of the temporary Fmoc group:* Piperidine (5 mL, 20% in DMF) was added to 0.1 mmol of Fmoc-peptide bound to the solid support. Nitrogen was bubbled through the mixture during 15 min. The resin was filtered and it was washed with DMF (3 x 5 mL x 3 min). The deprotection was verified performing a TNBS test on a small fraction of the resin.

*Coupling of the amino acids:* DIEA (3 mL, 0.195M in DMF) is added to the corresponding amino acid (0.4 mmol) dissolved in 2 mL of a 0.2 M HOBt and 0.2 M HBTU solution in DMF. The resulting mixture was stirred for 2 min and then added to the resin. Nitrogen is made to pass through the resulting mixture during 45 min or until it is observed that the coupling is complete, by the TNBS test in a small aliquot of the resin. The resin was filtered and washed with DMF (3 x 5 mL x 3 min). The synthesis continues with the following cycle of deprotection/coupling in a manner similar to that described.

*Selective deprotection of the side chain of the Lys(Alloc) residue:* Once the peptide sequence has been completed, the selected deprotection of the lysine is carried out using the following process: 0.1 mmol of the peptide bound to the resin is treated with Pd(PPh₃)₄ (1 equiv) and morpholine (190 equiv) in 2% H₂O/CH₂Cl₂ (5 mL) during 5 h. The resin was filtered and washed with DMF (3 x 5 mL x 3 min), diethydilithiocarbamate (DEDTC, 25 mg in 5 mL of DMF, 2 x 5 mL x 2 min), DMF (3 x 5 mL x 2 min) and CH₂Cl₂ (3 x 5 mL x 2 min).

*Deprotection*/*release of the resin:* The synthesized peptide still bound to the resin was dried under a nitrogen current and was treated in cold with the deprotection cocktail (50 µL CH₂Cl₂, 25 µL water 25 µL TIS and TFA until 1 mL for 40 mg of resin). The peptides containing cysteins were deprotected using the specific alternative cocktail: 25 µL EDT, 25 µL water 10 µL TIS and TFA until 1 mL, for each 40 mg of resin. The suspension resulting from adding the deprotection mixture to the resin was stirred during 2 h at ambient temperature. The resin was separated and the filtrates were added on ethyl ether in an ice bath (10 mL of ether for each mL of cocktail). After 10 min, the mixture was centrifuged and the decanted solid is washed with cold ether and dried under an argon current. The precipitate was dissolved in 2 mL of a mixture of acetonitrile/water 1:1 and it was analysed by HPLC in reverse phase using the aforementioned conditions. The main product in all the synthesis was identified as the desired peptide by mass spectrometry. The fractions isolated in preparatory HPLC were lyophilized and kept at -20°C.

### EXAMPLE 3.- Synthesis of the fluorescent hybrids.

Once the synthesis was completed, the Fmoc group was eliminated from the N-terminal residue following the standard process. The resin was washed and added to a solution of DIEA (0.5 M, 6 equiv) in DMF and fluorescein isothiocyanate (FITC, 4 equiv). The resulting mixture was stirred during 1 h at ambient temperature. The resin was washed with DMF (2 x 5 mL) and the Alloc group was deprotected following the aforementioned process. The resulting peptidyl-resin was subjected to the standard deprotection/release and purification process to give compounds **1B-5B**. Alternatively, the peptidyl-resin was coupled to the aminotripyrrole unit to synthesize derivatives **1A-5A**. For the couplings with the tripyrroles the resin was resuspended in DMF (1 mL) and stirred for 30 min to conveniently expand it. The DMF was filtered and DIEA (0.5 M in DMF, 8 equiv), DMAP (2 equiv in DMF) and *N,N*'=disuccinimidyl bicarbonate (15 equiv) was added to the resin. The resulting mixture was stirred for 2 h. The resin was washed with DMF and the aminotripyrrole 7 was added in DMF (4 equiv), DIEA (8 equiv, 0.5M in DMF) and DMAP (2 equiv). The reaction mixture was stirred during 2h and was then washed with DMF (2 x 5 mL x 2 min) and ethyl ether (2 x 5 mL x 2 min). The deprotection/release following standard conditions gave rise to the expected conjugates that were purified by HPLC in reverse phase and identified by mass spectrometry as detailed above.

### EXAMPLE 4.- Synthesis of hybrid 5A.

This compound was obtained by coupling of the corresponding peptide that included a cisterna, with the cystein-ortaarginine peptide previously activated by reaction with DTNB (Ellman reagent). The coupling reaction was carried out in 100 mM Tris-HCl buffer, 1M NaCl, pH 7.5 stirring the mixture at ambient temperature for 45 min. The main product is purified by HPLC following the standard conditions and was identified by mass spectrometry how the heterodynamic hybrid containing the desired disulfide bridge.

### Preliminary cytotoxicity studies

Preliminary cytotoxicity studies have been performed using HeLa 229 cells, to verify the possible effect of the presence of octaarginine chain on the biological activity. These studies have been performed following an incubation during 48 hours and they were later evaluated by cell staining by crystal violet and later acetylation.

It has been verified that effectively the presence of octaarginine in tripyrrole compounds significantly increases the percentage of cell growth inhibition. As gathered in the following table, the most active compound is **1A**, which is any all cases significantly more efficient that the analogue **10,** which does not have the octaarginine chain.

**Table 1**

| **compound** | **Concentration (µM)** | **% Inhibition of the cellular growth** |
|---|---|---|
| **9** | 176.5 | 4±3 |
| **10** | 4.53 | 1±3 |
| **1A** | 2.63 | 20±2 |
| **1B** | 5.87 | 1±2 |

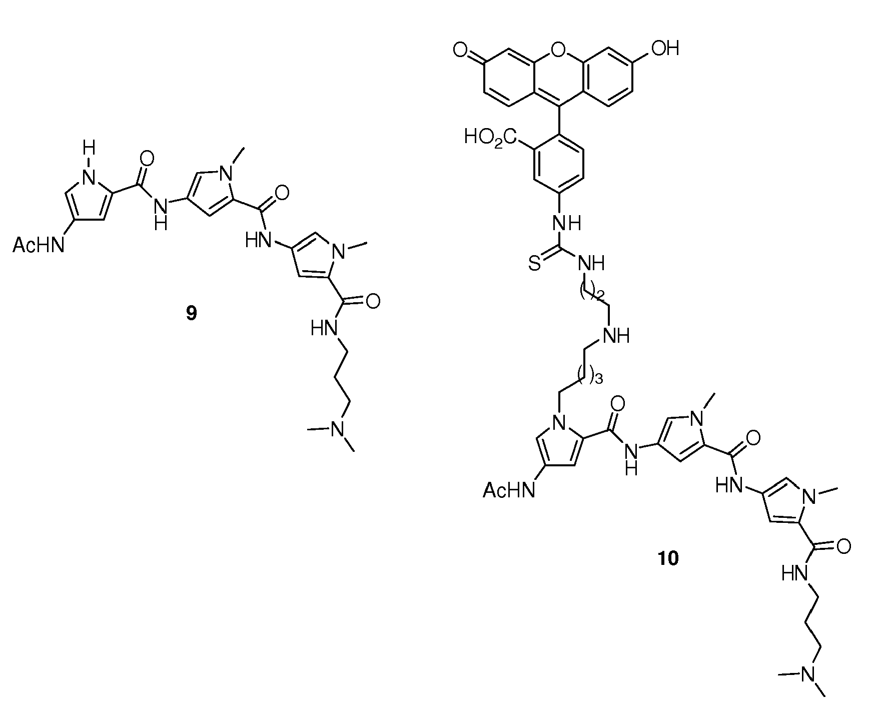

## Claims

1. Compound comprising a tripyrrole group bound to an octaarginine ((Arg)₈) by means of a spacer comprising, at least, one lysine (Lys).

2. Compound according to claim 1, which further comprises a fluorophore group.

3. Compound according to anyone of claims 1 to 2, where the tripyrrole has the formula (I): 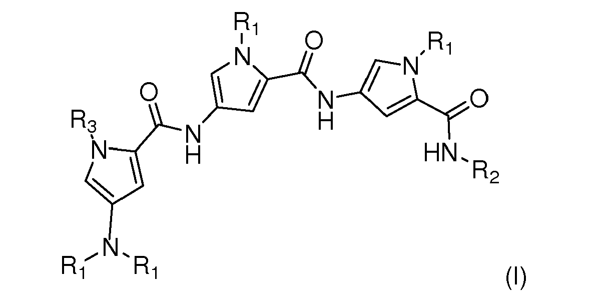 R1 is the same or different and is independently selected from the list comprising H, an alkyl group (C₁-C₆) or an acyl group;
R2 is a substituted alkyl group of general formula (IIA): 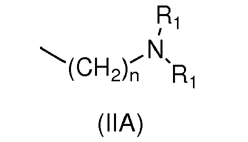 or of general formula (IIB): 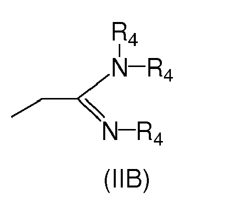 R3 is a substituted alkyl group of general formula (III): 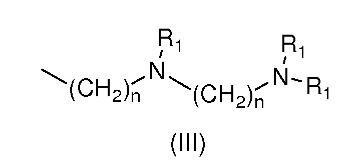 where n takes values between 1 and 6.

4. Compound according to claim 3, where the tripyrrole is: 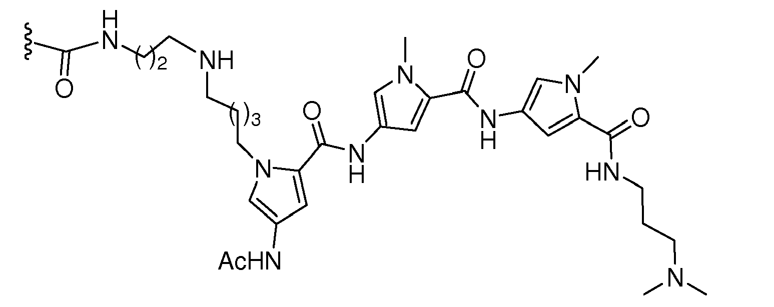 where 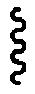 represents the binding site with the lysine of the spacer.

5. Compound according to anyone of claims 1 to 4, where the spacer in addition to the lysine comprises 6-aminohexanoic acid (Ahx).

6. Compound according to claim 5, where the spacer has the formula (Ahx-Lys-Ahx):

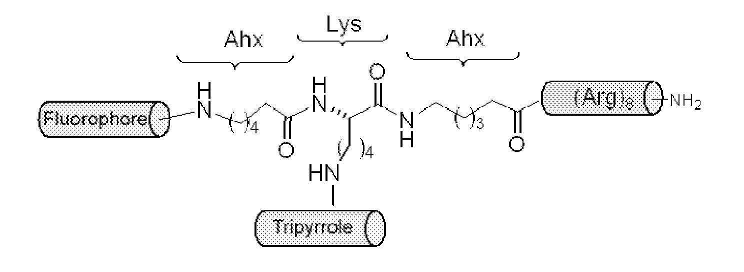

7. Compound according to anyone of claims 2 to 6, where the fluorophore is fluorescein.

8. Method of obtainment of a compound according to anyone of claims 1 to 7, comprising the following steps:
a. synthesis of the peptide in solid phase,
b. coupling to the tripyrrole fragment,
c. deprotection of side chains and separation of the resin.

9. Use of the compound according to anyone of claims 1 to 7, for the preparation of a drug.

10. Use of the compound according to claim 9, for the preparation of a drug for the treatment of cancer.

11. Use of the compound according to claim 9, for the preparation of a drug for the treatment of microbial diseases.

12. Use of the compound according to anyone of claims 1 to 7, to efficiently interfere with cellular processes at DNA level.
